(19) Europäisches Patentamt / European Patent Office / Office européen des brevets

(11) **EP 2 264 071 A1**

(12) **EUROPEAN PATENT APPLICATION**
published in accordance with Art. 153(4) EPC


(43) Date of publication:
**22.12.2010 Bulletin 2010/51**

(21) Application number: **09718974.0**

(22) Date of filing: **13.03.2009**

(51) Int Cl.:
***C07K 16/28*** *(2006.01)* ***A61K 39/395*** *(2006.01)*
***C12Q 1/04*** *(2006.01)* ***C12P 21/08*** *(2006.01)*

(86) International application number:
**PCT/JP2009/054842**

(87) International publication number:
**WO 2009/113649 (17.09.2009 Gazette 2009/38)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **14.03.2008 JP 2008066821**
**15.01.2009 JP 2009007053**



(71) Applicant: **Medinet., Co. Ltd.**
**Yokohama-shi, Kanagawa 222-0033 (JP)**

(72) Inventors:
- **YAMASHIRO, Hiromichi**
  **Tokyo 158-0096 (JP)**
- **TADAKI, Toshimasa**
  **Tokyo 158-0096 (JP)**

(74) Representative: **Carpmaels & Ransford**
**One Southampton Row**
**London**
**WC1B 5HA (GB)**


(54) **ANTIBODY HAVING AN IMMUNE-ENHANCEMENT FUNCTION**


(57) The present invention provides a monoclonal antibody against FLJ32028, which binds specifically to the surfaces of regulatory T-cells (Treg) and especially to the surfaces of induced Treg. This monoclonal antibody can be used for a Treg-removal method or a Treg-removal apparatus. This monoclonal antibody can also be used as a drug that improves cell-proliferation function, an immune-enhancement function, or especially for cancer treatment.


FIG.1

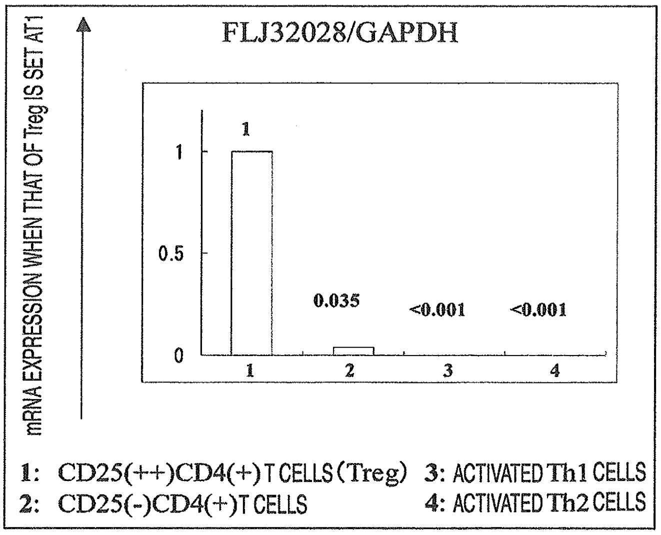


EP 2 264 071 A1

<110> MEDINET Co.,Ltd.

<120> Antibodies having functions enhancing immune response

<130> W5174-000000

<150> JP 2008-066821
<151> 2008-03-14

<150> JP 2009-007053
<151> 2009-01-15

<160> 4

<210> 1
<211> 552
<212> DNA
<213> Homo sapiens

<400> 1
```
atgcaggctc cccgcgcagc cctagtcttc gccctggtga tcgcgctcgt tcccgtcggc    60
cggggtaatt atgaggaatt agaaaactca ggagatacaa ctgtggaatc tgaaagacca   120
aataaagtga ctattccaag cacatttgct gcagtgacca tcaaagaaac attaaatgca   180
aatataaatt ctaccaactt tgctccggat gaaaatcagt tagagtttat actgatggtg   240
ttaatcccat tgattttatt ggtcctctta cttttatccg tggtattcct tgcaacatac   300
tataaaagaa aaagaactaa acaagaacct tctagccaag gatctcagag tgctttacag   360
acatatgaac tgggaagtga aaacgtgaaa gtccctattt ttgaggaaga tacaccctct   420
gttatggaaa ttgaaatgga agagcttgat aaatggatga acagcatgaa tagaaatgcc   480
gactttgaat gtttacctac cttgaaggaa gagaaggaat caaatcacaa cccaagtgac   540
agtgaatcct aa                                                       552
```

<210> 2
<211> 183
<212> PRT
<213> Homo sapiens

<400> 2
```
Met Gln Ala Pro Arg Ala Ala Leu Val Phe Ala Leu Val Ile Ala Leu
                5                   10                  15
Val Pro Val Gly Arg Gly Asn Tyr Glu Glu Leu Glu Asn Ser Gly Asp
            20                  25                  30
Thr Thr Val Glu Ser Glu Arg Pro Asn Lys Val Thr Ile Pro Ser Thr
        35                  40                  45
Phe Ala Ala Val Thr Ile Lys Glu Thr Leu Asn Ala Asn Ile Asn Ser
    50                  55                  60
Thr Asn Phe Ala Pro Asp Glu Asn Gln Leu Glu Phe Ile Leu Met Val
65                  70                  75                  80
Leu Ile Pro Leu Ile Leu Leu Val Leu Leu Leu Leu Ser Val Val Phe
                85                  90                  95
Leu Ala Thr Tyr Tyr Lys Arg Lys Arg Thr Lys Gln Glu Pro Ser Ser
            100                 105                 110
Gln Gly Ser Gln Ser Ala Leu Gln Thr Tyr Glu Leu Gly Ser Glu Asn
        115                 120                 125
Val Lys Val Pro Ile Phe Glu Glu Asp Thr Pro Ser Val Met Glu Ile
    130                 135                 140
Glu Met Glu Glu Leu Asp Lys Trp Met Asn Ser Met Asn Arg Asn Ala
145                 150                 155                 160
Asp Phe Glu Cys Leu Pro Thr Leu Lys Glu Glu Lys Glu Ser Asn His
                165                 170                 175
```

```
Asn Pro Ser Asp Ser Glu Ser
            180

<210> 3
<211> 54
<212> DNA
<213> Artificial sequence
<220>
<223> PCR primer

<400> 3
aataagcttg ccaccatgca ggctccccgc gcagccctag tcttcgccct ggtg        54

<210> 4
<211> 41
<212> DNA
<213> Artificial sequence
<220>
<223> PCR primer

<400> 4
ccatctagat taggattcac tgtcacttgg gttgtgattt g                      41
```

SEQUENCE LISTING

<110> MEDINET Co.,Ltd.
<120> Antibodies having functions enhancing immune response
<130> W5174-000000
<150> JP 2008-066821
<151> 2008-3-14
<150> JP 2009-007053
<151> 2009-1-15
<160> 4

<210> 1
<211> 552
<212> DNA
<213> human
<400> 1
atgcaggctc cccgcgcagc cctagtcttc gccctggtga tcgcgctcgt tcccgtcggc 60
cggggtaatt atgaggaatt agaaaactca ggagatacaa ctgtggaatc tgaaagacca 120
aataaagtga ctattccaag cacatttgct gcagtgacca tcaaagaaac attaaatgca 180
aatataaatt ctaccaactt tgctccggat gaaaatcagt tagagtttat actgatggtg 240
ttaatcccat tgatttattt ggtcctctta cttttatccg tggtattcct tgcaacatac 300
tataaaagaa aaagaactaa acaagaacct tctagccaag gatctcagag tgctttacag 360
acatatgaac tgggaagtga aaacgtgaaa gtccctattt ttgaggaaga tacaccctct 420
gttatggaaa ttgaaatgga agagcttgat aaatggatga acagcatgaa tagaaatgcc 480
gactttgaat gtttacctac cttgaaggaa gagaaggaat caaatcacaa cccaagtgac 540
agtgaatcct aa 552

<210> 2
<211> 183
<212> PRT
<213> human
<400> 2
Met Gln Ala Pro Arg Ala Ala Leu Val Phe Ala Leu Val Ile Ala Leu
5 10 15
Val Pro Val Gly Arg Gly Asn Tyr Glu Glu Leu Glu Asn Ser Gly Asp
20 25 30
Thr Thr Val Glu Ser Glu Arg Pro Asn Lys Val Thr Ile Pro Ser Thr
35 40 45
Phe Ala Ala Val Thr Ile Lys Glu Thr Leu Asn Ala Asn Ile Asn Ser
50 55 60
Thr Asn Phe Ala Pro Asp Glu Asn Gln Leu Glu Phe Ile Leu Met Val
65 70 75 80
Leu Ile Pro Leu Ile Leu Leu Val Leu Leu Leu Leu Ser Val Val Phe
85 90 95
Leu Ala Thr Tyr Tyr Lys Arg Lys Arg Thr Lys Gln Glu Pro Ser Ser
100 105 110
Gln Gly Ser Gln Ser Ala Leu Gln Thr Tyr Glu Leu Gly Ser Glu Asn
115 120 125
Val Lys Val Pro Ile Phe Glu Glu Asp Thr Pro Ser Val Met Glu Ile
130 135 140
Glu Met Glu Glu Leu Asp Lys Trp Met Asn Ser Met Asn Arg Asn Ala
145 150 155 160
Asp Phe Glu Cys Leu Pro Thr Leu Lys Glu Glu Lys Glu Ser Asn His
165 170 175
Asn Pro Ser Asp Ser Glu Ser
180

<210> 3
<211> 54
<212> DNA
<213> Artificial sequence
<220> PCR primer

```
<400> 3
aataagcttg ccaccatgca ggctccccgc gcagccctag tcttcgccct ggtg        54

<210> 4
<211> 41
<212> DNA
<213> Artificial sequence
<220> PCR primer
<400> 4
ccatctagat taggattcac tgtcacttgg gttgtgattt g                      41
```

**Description**

TECHNICAL FIELD

The present invention relates to a new antibody which enhances immunity. In addition, the present invention relates to applications such as a medicament using the antibody.

BACKGROUND ART

The immune system has a key role in protecting the human body from external non-self materials, pathogens, and the like; when its mechanism is impaired or becomes dysfunctional, there occur problems such as the appearance of various disorders and increased risk to serious diseases.

For example, allergy and autoimmune diseases develop based on excessive immune reaction to an object which should be immunologically tolerated in nature.

In contrast to the excessive immune reaction, tumor cells during the growth, propagation and metastasis of cancer has a (so-called tumor immunity) mechanism which suppresses the immune system and prevents the immune system against tumor cells, i.e., "an immuno-suppressive function", which thereby allows the propagation and metastasis of cancer to be repeated.

Recently, many studies have been conducted on the mechanism of the immunity-suppression and the mechanism has been gradually elucidated.

Among the elucidated mechanisms, attention has been given in recent years particularly to a mechanism for a method of cancelling a state of "immuno-suppression" around tumor tissue.

Tumor tissue is thought to reduce immune function around the tumor tissue by producing immuno-suppressive factor such as TGF-β, IL-10, and PGE2, and thereby to avoid the cancer tissue from being attacked by body's own immune cells.

Accordingly, cancer treatment is expected to be improved by cancelling the state of immuno-suppression to enhance the immune function; various studies have been conducted.

$CD4^+CD25^+FoxP3^+$ cells and the like are mainly exemplified as a cell group responsible for the immuno-suppression and known as regulatory T cells (hereinafter sometimes referred to as "Treg"). The Treg is roughly classified into naturally occurring Treg (hereinafter sometimes referred to as "nTreg") and induced Treg (hereinafter sometimes referred to as "iTreg").

nTreg are cells which differentiate principally within the thymus and are present in an amount of about 5% in the population of peripheral blood mononuclear cells. For example, a mechanism exists in which allergy, autoimmune disease, and the like are typically avoided by the action of the nTreg.

In contrast, iTreg are presumed to be Treg which differentiate in secondary lymphoid tissues (peripheral tissues) and are present only in a particular environment such as around tumor tissue; the iTreg are thought to act on around tumor tissue to cause an immuno-suppressive state. Thus, some research has been started as one of researches on methods for treating cancer for antibodies thereto or for markers for recognizing these cells, and the like.

Methods for cancelling the immuno-suppression due to Treg include, for example, (1) a method using anti-CD25 antibody alone or in conjugation with diphtheria toxin or the like (Non-Patent Document 1), (2) a method using anti-GITR antibody (Non-Patent Document 2), and a method using anti-CTLA-4 antibody (Non-Patent Document 3).

However, the method in (1) above results in the depletion of all CD25-positive cells and in the elimination of activated lymphocytes other than Treg as well as the depletion of Treg because of CD25-positive cells being also cells responsible for activation. For the method in (2), the treatment showed effective response in mice model (Non-Patent Document 2), but there is no definite data as being effective in human study. The method using anti-CTLA-4 antibody in (3) has a certain effect against melanoma (Non-Patent Document 3), but its effectiveness against other cancer species remains uncertain.

Patent Document 1 describes that FLJ32028 protein was isolated as a protein associated with B cell chronic lymphocytic leukemia and this protein will be able to provide a diagnostic marker for B cell chronic lymphocytic leukemia and that an antibody to the protein is used for diagnosing B cell chronic lymphocytic leukemia. However, Patent Document 1 describes no relation of FLJ32028 protein to Treg. Patent Document 2 describes that folate receptor 4 is highly expressed on the surface of Treg and Treg will be able to detectby recognition of this receptor as a marker using an antibody to the receptor. However, Patent Document 2 describes no relation of FLJ32028 protein to Treg.

PATENT DOCUMENT 1
International Publication No. WO 2004/110369 Pamphlet
PATENT DOCUMENT 2

JP2006-304740A
NON-PATENT DOCUMENT 1
Dannull J, et al., J Clin Invest. 2005 Dec; 115 (12): 3623-33
NON-PATENT DOCUMENT 2
Shimizu J, et al., Nature Immunology 3, 135-142 (01 Feb 2002)
NON-PATENT DOCUMENT 3
Phan GQ, et al., Proc. Natl. Acad. Sci. U.B.A. 100, 372

DISCLOSURE OF THE INVENTION

PROBLEMS TO BE SOLVED BY THE INVENTION

As described above, the depletion of iTreg is thought to be effective in treating cancer, but a specific method therefor is not yet established.
Thus, an object of the present invention is to provide an antibody capable of specifically depleting Treg and the use thereof.

MEANS FOR SOLVING THE PROBLEMS

To overcome the above problems, the present inventors have verified the presence of a molecule capable of providing a marker specific for the Treg and have elucidated that FLJ32028 is a molecule expressed specifically on Treg, particularly on the surface of iTreg. The present inventors have further obtained a monoclonal antibody to this molecule, thereby accomplishing the present invention.
Thus, the present invention relates to the following [1] to [19]:

[1] An antibody recognizing a transmembrane molecule, FLJ32028, wherein the antibody specifically binds to regulatory T cells;
[2] The antibody according to item [1] above, wherein the antibody binds to the transmembrane molecule FLJ32028 specifically expressed on a surface of the regulatory T cells;
[3] The antibody according to item [1] or [2] above, wherein the regulatory T cells are induced regulatory T cells;
[4] The antibody according to any of items [1] to [3] above, wherein the antibody inhibits differentiation of precursor cells of induced regulatory T cell into the induced regulatory T cells;
[5] The antibody according to any of items [1] to [4] above, wherein the antibody is a monoclonal antibody;
[6] The antibody according to item [5] above, wherein the monoclonal antibody is produced from a hybridoma internationally deposited under the Budapest Treaty and given the accession number FERM ABP-11100;

[7] A depletion method for regulatory T cells, comprising binding the regulatory T cells to the antibody according to any of items [1] to [6] above to deplete the regulatory T cells;
[8] The depletion method according to item [7] above, wherein the regulatory T cells are induced regulatory T cells;
[9] A depletion device for regulatory T cells, comprising the antibody according to any of items [1] to [6] above;
[10] The depletion device according to item [9] above, wherein the regulatory T cells are induced regulatory T cells;
[11] A medicament comprising the antibody according to any of items [1] to [6] above;
[12] The medicament according to item [11] above for cancelling immuno-suppression due to regulatory T cells;
[13] The medicament according to item [11] or [12] above for proliferating cells whose proliferation has been suppressed by regulatory T cells;
[14] The medicament according to any of items [11] to [13] above for enhancing immune function;
[15] The medicament according to any of items [11] to [14] above, wherein the regulatory T cells are induced regulatory T cells;

[16] A marker for detection of regulatory T cells, comprising a transmembrane molecule, FLJ32028 protein, or a fragment thereof;
[17] The marker for detection of regulatory T cells according to item [16], wherein the regulatory T cells are induced regulatory T cells;
[18] A detection method for regulatory T cells in subject cells suspected of expressing a transmembrane molecule, FLJ32028, on the cell surface thereof, comprising contacting the subject cells with the antibody according to any one of claims 1 to 6 to detect expression of the transmembrane molecule FLJ32028 protein on the surface of the subject cells; and

[19] The detection method according to item [18] above, wherein the regulatory T cells are induced regulatory T cells.

ADVANTAGES OF THE INVENTION

The antibody of the present invention recognizes FLJ32028 expressed on the surface of iTreg; thus, it can target iTreg emerging around tumor tissue to suppress the function thereof. That is, the antibody can cancel a state of immuno-suppression around tumor tissue, for example, to enhance immune function. In addition, the antibody of the present invention can inhibit the differentiation of precursor cells of induced regulatory T cells into the induced regulatory T cells.
This enables the provision of effective treatment of cancer by using the antibody of the present invention as an immunostimulating agent or a cancer therapeutic agent or by using it in cell culture employed when performing cancer treatment by immuno-cell therapy.

BRIEF DESCRIPTION OF THE DRAWINGS

Fig. 1 is a graph showing the results of real time PCR for a FLJ32028 molecule using $CD4^+CD25^-$ or $CD4^+CD25^+$ cell-derived cDNA as a template;

Fig. 2 is a histogram confirming the antigen specificity of anti-FLJ32028 antibody by transiently expressing a plasmid vector encoding a FLJ32028 molecule or a control vector on CHO cells;

Fig. 3 is a graph showing the results of attaching a magnetic bead to anti-FLJ32028 antibody, reacting the complex with human PBMC, and removing the FLJ32028 molecule, followed by measuring cell proliferation using anti-CD3 antibody;

Fig. 4 is a graph showing cytotoxic activity when K562 was mixed with PBMC treated as described in Fig. 3 in a mixing ratio of 1:1;

Fig. 5 is a graph showing the results of FACS analysis of intracellular IFN-γ production after recovering and PMA- and ionomycin-retreating PBMC treated as described in Fig. 3;

Fig. 6 is a graph showing cell proliferation measured by immobilizing anti-FLJ32028 antibody on a plate or adding anti-FLJ32028 antibody to a culture medium in stimulating human PBMC with anti-CD3 antibody;

Fig. 7 is a set of graphs showing the results of FACS analysis using various antibodies 6 to 7 days after the start of culture of human $CD4^-CD25^-$ cells under conditions of inducing the cells into iTreg;

Fig. 8 is a graph showing the percentage of cancelled immuno-suppression when a control antibody or anti-FLJ32028 antibody was added in adding iTreg in allo-MLR;

Fig. 9 is a graph showing the intensity of FoxP3 expression on the cells obtained when culture was performed by adding the antibody of the present invention in inducing iTreg;

Fig. 10 is a set of graphs showing the cytotoxic activity of effector cells against tumor cells when cells obtained after performing culture by adding the antibody of the present invention in inducing iTreg were co-cultured with the effector cells and the tumor cells;

Fig. 11 is a graph showing the cytotoxic activity of effector cells against tumor cells when cells obtained after performing culture by adding the antibody of the present invention to iTreg after induction were co-cultured with the effector cells and the tumor cells; and

Fig. 12 is a graph showing a change in the tumor mass when the antibody of the present invention was administered to immuno-deficient mice in which U937 was implanted.

BEST MODE FOR CARRYING OUT THE INVENTION

The embodiments of the present invention will be described below.

Antibody to FLJ32028

The antibody to a FLJ32028 molecule according to the present invention (hereinafter sometimes referred to as "the antibody of the present invention") is first described.
The antibody of the present invention is an antibody recognizing a surface molecule of Treg, FLJ32028, and can recognize Treg, particularly induced Treg to specifically bind to induced Treg.
The antibody of the present invention is an antibody recognizing the FLJ32028 molecule. As described above, FLJ32028 is Treg-specifically expressed; thus, the antibody of the present invention has the characteristic of specifically binding to Treg.

The antibody of the present invention may be any antibody provided that it is a cloned immunoglobulin antibody; the animal species from which the antibody is derived, the type and subclass of the immunoglobulin, and the production method for the antibody do not matter. It also encompasses fragments of the antibody in which the binding sites for immune reaction are kept, modulators of the fragments, modulators of the antibody itself, a chimeric antibody in which two antibodies including the antibody are bound, and a humanized antibody thereof. The antibody of the present invention may be an antiserum, a polyclonal antibody, or a monoclonal antibody, and is particularly preferably a monoclonal antibody.

FLJ32028 is a well-known protein having a molecular weight of about 20 kDa, and has been identified as single-pass transmembrane protein 154 (TMEM154) or HGNC ID26489. However, the ligand therefor is not found at the moment. It has been deposited in GenBank under the accession number AK056590 (Nat. Genet. 36(1): 40-45 (2004)). The gene encoding FLJ32028 protein is as represented by SEQ ID NO: 1 of the Sequence Listing; the coding gene for FLJ32028 protein is positioned between nucleotides 1 and 552. The amino acid sequence of FLJ32028 protein is as represented by SEQ ID NO: 2 of the Sequence Listing. It is probable that its part outside the cell membrane is positioned between residues 23 and 75. The present inventors have now found that the molecule is expressed on Treg, particularly on iTreg, and rendered the iTreg capable of being effectively depleted using an antibody thereto, preferably a monoclonal antibody thereto, thereby accomplishing the present invention.

Specifically, the FLJ32028 molecule can be obtained by selecting the cell group of regulatory T cells from peripheral blood cells, removing $CD4^+CD25^-$ cells, helper T cells 1 (Th1), and helper T cells 2 (Th2) therefrom to provide $CD4^+CD25^+$ cells, and analyzing proteins expressed on the surface of the cells.
In this case, heretofore known methods can be used as needed; examples thereof include an SAGE technique and a gene differential display technique; however, for example, a subtraction technique can be used to suitably identify the molecule.

Specifically, the subtraction technique involves preparing a cell subpopulation believed to be regulatory T cells ($CD4^+CD25^+$) and groups of Th1 and Th2 cells for subtracting genes therefrom from healthy donor peripheral blood mononuclear cells, preparing RNA and cDNA from the respective groups of cells, and performing gene subtraction by hybridization.
The Treg-specific cDNA fragments obtained by the subtraction are each inserted into a vector for TA cloning and sequenced using a DNA sequencer. A BLAST search can be carried out based on the determined base sequence to identify a Treg-specifically expressed molecule.

The antibody of the present invention can be properly produced using a heretofore known technique. For example, an animal may be immunized with FLJ32028 antigen protein to provide its serum, or, for example, a hybridoma prepared by cell fusion using a fusion enhancing reagent such as polyethylene glycol may be used to produce the antibody, or DNA predicted from the amino acid sequence of the antibody may be used to produce the antibody by a production method employing gene engineering or the like.
The hybridoma producing the monoclonal antibody of the present invention can be obtained by a method according to a well-known method, that is, by immunizing an animal such as a mouse and a rat with an immunogen, fusing B cells of the immunized animal and myeloma cells, and selecting a cell line producing the monoclonal antibody of the present invention from among the resulting hybridomas.

The immunogen with which an animal such as a mouse and a rat is immunized may be used in various forms. Examples thereof include FLJ32028 protein or its peptide fragment, a vector in which the gene encoding FLJ32028 or its fragment is introduced, iTreg cells, and a transfectant expressing FLJ32028. When the peptide fragment is used, the animal is preferably immunized with a peptide part outside the cell membrane. As one example, a method performing the immunization using cDNA encoding FLJ32028 is described below in detail.

cDNA can be prepared from human peripheral blood lymphocytes and used as a template to amplify the full length of the gene employing gene-specific primers, followed by the insertion thereof into a mammalian expression vector to clone FLJ32028. The insertion of the full length sequence into the vector allows FLJ32028 to correctly form the tertiary structure thereof and be expressed on the cell membrane when the immune animal is immunized with the DNA. Thus, an antibody can be obtained which recognizes a tertiary structure of FLJ32028 exposed outside the cell membrane.

Production of Monoclonal Antibody Specific to FLJ32028

An immunized animal (e.g., mouse) is immunized with the identified FLJ32028 molecule. A mixture of a vector having the FLJ32028 molecule-encoding gene sequence inserted and an immunostimulator such as a gold colloid is introduced into an 8- to 10-week old female Balb/c mouse using a gene gun, and the mouse is housed for about 2 to 3 months. An increase in the antibody value to FLJ32028 can be confirmed by the following procedure.

1) The above gene-inserted vector is introduced into suitable cells (e.g., CHO cells) to prepare cells in which FLJ32028 is transiently expressed.
2) These cells are mixed with a serum (containing a polyclonal antibody to FLJ32028) collected from the immune animal.
3) FCM analysis is performed using a fluorescently labeled anti-mouse polyclonal antibody (secondary antibody).

As described above, the antibody value can be evaluated using the cells in which FLJ32028 is transiently expressed to confirm whether or not an antibody recognizing a tertiary structure of FLJ32028 exposed outside the cell membrane has been obtained.
After confirming a strong increase in the antibody value, the cells (e.g., CHO cells) transiently expressing FLJ32028 are intraperitoneally injected in the immune animal for final immunization. Three to five days after the final immunization, spleen cells of the immunized animal are taken out and fused with myeloma cells (e.g., SP2/0 mouse myeloma cells) using a polyethylene glycol (PEG1500 or the like) to prepare hybridomas. A hybridoma producing a desired antibody to FLJ32028 can be selected from the group of the hybridoma cell lines prepared, by mixing the cells transiently expressing FLJ32028 with a hybridoma culture supernatant, followed by performing FCM analysis using a fluorescently labeled anti-mouse polyclonal antibody (secondary antibody).
One hybridoma cell line thus obtained was designated as Mouse-Mouse hybridoma FLJ32028-41 and deposited in International Patent Organism Depositary, National Institute of Advanced Industrial Science and Technology (Higashi 1-chome 1-banchi 1 chuo dai-6, Tsukuba City, Ibaraki, Japan) March 6, 2008 (accession number: FERM P-21522). The deposit was transferred to the international deposit according to the Budapest Treaty February 26, 2009 (accession number: FERM ABP-11100). This hybridoma can be used to easily provide the monoclonal antibody of the present invention.
The antibody of the present invention thus obtained specifically recognizes FLJ32028 expressed on the Treg surface, particularly on the iTreg surface; thus, this enables the antibody to specifically bind to, and deplete, iTreg probably present in the blood or around tumor tissue.
The antibody of the present invention can be used to improve the proliferative potential of cells for culture used in immuno-cell therapy by depleting Treg present before the culture, and can also be administered to the periphery of tissues such as tumor tissue to cancel immuno-suppression occurring around such tissues.

Depletion Method for Treg and Depletion Device for Treg

The Treg depletion method and Treg depletion device of the present invention involves, for example, binding the antibody of the present invention to a polymer compound such as beads and filling up a column or the like with them to effectively deplete Treg from blood.
As used herein, the polymer compound is selected from polymers not dissolving under contact with a body fluid and having such low toxicity as to do no harm under contact with blood cell components; examples thereof include polyurethane, polystyrene, polysulfone, polyvinyl chloride, acrylic resins, polyamide resins, phenoxy resin, urethane resin, fluorine-containing resins, silicon resins, cellulose resins, chitin, chitosan, agarose, and dextran. These polymer materials may be used alone or may constitute a copolymer, complex or mixture thereof.
Specific constitutional examples include beads attached to the antibody of the present invention, with which a column is filled up, for depleting Treg by passing blood therethrough.
The attachment of the antibody of the present invention to the polymer compounds is fixed by chemical bonding. Examples of the chemical bonding include covalent bonding, ionic bonding, and hydrophobic bonding; among these, covalent bonding is preferable because it enables sufficient fixation. To fix the attachment by covalent bonding, the polymer compounds preferably have appropriate functional groups; preferably, they have groups such as an amino group, a hydroxyl group, a carboxyl group, a thiol group, a glycidyl group, an isocyanate group, and a halogen group. Among others, a tosyl group, an epoxy group, and the like are suitably used, for example.
When filling up a column with the polymer compound and the antibody of the present invention are packed in a column, the shape thereof is not particularly limited; examples thereof include a film, a fiber, a hollow fiber, and a gel in addition to the bead.
The amount of fixation of the antibody of the present invention to the polymer compound is preferably 1 pmole to 10 moles per g of the polymer compound because an insufficient amount of the antibody of the present invention makes the depleting effect poor and an excessive amount thereof may cause steric hindrance because of the antibody molecules overlapping each other.
The Treg depletion method and Treg depletion device of the present invention thus constituted can efficiently deplete Treg.
Specifically, for example, when the collected blood passed through the device is used for cell culture for immuno-cell therapy, the culture efficiency is enhanced because Treg is depleted; for example, when the blood or other body fluids of a patient are extracorporeally circulated as in the case of dialysis, adopting the depletion method of the present invention, or returning the blood or other body fluids adapted to be passed through the depletion device into the patient's body enables the improvement of the patient's immune function, leading to an improved therapeutic efficiency.

Medicament Containing Antibody of the Present Invention

The medicament of the present invention is characterized by containing the antibody of the present invention. Treg can be depleted in advance from a group of cells using the antibody of the present invention to expect the following advantages.

1) The rate of cell proliferation can be enhanced because an inhibitor in the cell culture is cleared. That is, the function of enhancing the proliferation of cells whose proliferation has been suppressed by Treg can be exhibited, such as, for example, lymphocytes and cytokine producing cells.
2) Administration of the antibody into the body of a patient whose immune function is reduced enhances the general immune function of the patient and thereby can increase the effect of treating various diseases, enabling the antibody to exert a generally immune-enhancing function.
3) A state of immuno-suppression occurs around diseased tissues in diseases such as cancer; thus, the state of suppression can be cancelled to enhance the effect of treating cancer, the function of cancelling the immuno-suppression being exerted in cancer. Particularly, the antibody of the present invention can suppress the differentiation of precursor cells such as $CD4^{+}CD25^{-}$ T cells into iTreg to cancel the immuno-suppressing effect of Treg.

As described above, the function of 1) is suitable particularly for the immuno-cell therapy involving treatment using patient's own cells; the function of 2) can be used in a wide range of subjects, and is particularly expected to enhance the effect of preventing or treating a reduced immune function due to infection or the like; and the function of 3) is effective in treating cancer as noted above.

The above effects can be sufficiently expected even when the medicament of the present invention is used alone; however, of course, synergetic effects can also be expected when it is used in combination with a conventionally similarly used agent or medicament.

Specifically, for example, Treg can be efficiently depleted by adding the antibody of the present invention to blood before cell proliferation, followed by culturing the cells, or by allowing the antibody of the present invention to contact around affected tissues.

For example, when the antibody is used to improve the proliferation function of cells as described in the above 1), in culturing patient's own cells used in immuno-cell therapy, for example, Treg may be depleted by adding the antibody to patient's blood in collecting the blood therefrom, or the culture may be started after adding the antibody early in the cell culture step. The addition amount of the antibody of the present invention can vary depending on the addition method and addition time thereof; however, it is typically 1 μg/ml to 50 μg/ml.

The medicament may be composed of the antibody of the present invention alone or its proper combination with another selected cytokine, agent, or the like.

When used for treatment, the medicament of the present invention may also be employed alone or in combination with another medicament; for example, it can be used in combination with an anti-cancer agent or with cells or a cell vaccine used in immuno-cell therapy.

The treatment of cancer using an anti-cancer agent is directed toward shrinking the cancer by the attack of cancer tissue by the anti-cancer agent; however, some side effects often also impair immune function which a patient originally has. In such a case, the medicament of the present invention is used in combination therewith to improve immune function, enabling the expectation of a synergistic effect combining a therapeutic effect of the anti-cancer agent therewith. That is, the medicament of the present invention can cancel immuno-suppression around the tissue and further promote the activation of immunocompetent cells, resulting in an improved therapeutic effect.
The anti-cancer agent is not limited to that of a particular type; those of various types can be used in combination therewith.

In the case of use in applications of the above 2) and 3), the antibody of the present invention and a substance having cytotoxic activity (e.g., a substance having an anticancer effect) are bound for use to allow the administered antibody to specifically bind to Treg to cause the substance having cytotoxic activity bound to the antibody to act on Treg to enable Treg to be specifically depleted. The adoption of such a method cancels immuno-suppression in the body, improves immune function, and is expected to enhance the effect of treatment used in combination.

For combined use in immuno-cell therapy, cultured cells may again not well function around tumor tissue which has become immuno-suppressed; thus, the cancer therapeutic agent of the present invention can be used in combination therewith to improve the therapeutic effect thereof.

The combined immuno-cell therapy can use LAK (lymphokine activated killer) cells, dendritic cells, NK cells, or various other cells.

Depending on the method of use, the dose of the medicament of the present invention may be on the order of about 0.0001 to 30 mg/kg in both cases of single use and combined use; the dose of such order can improve the therapeutic efficiency thereof.

The method for administering the medicament may be, for example, intravenous, intradermal, or subcutaneous injection, or injection into a lymph node. The medicament may be directly injected into an affected area; for example, it may be administered by endoscopically or percutaneously inserting a needle. In addition, the medicament may be injected through an artery in the vicinity of an affected area; for example, it may be administered via an arterial catheter selectively inserted into a cancer-feeding blood vessel.

As for administration timing, methods are possible such as a method involving: pre-administering the medicament of the present invention before the day of administration of a medicament such as an anti-cancer agent enhancing immune function and cultured cells for immuno-cell therapy; administering the former medicament on the day of administration of the latter medicament/the cells (concomitant administration); or administering the former medicament between the days of administration of the latter medicament/the cells.

As for the administration interval of the medicament of the present invention, the administration thereof on the order of every two weeks (concomitant with the administration of the cells) can be expected to have a sustained effect; however, besides that, it may be administered at an interval such as once a week or once a month in accordance with the disease and the combined medicament.

Marker for Detection of Regulatory T Cell. Comprising Transmembrane Molecule FLJ32028_ Protein or Fragment Thereof

The marker for detecting regulatory T cells according to the present invention will be described.
The marker for detecting regulatory T cells according to the present invention is a marker comprising a transmembrane molecule, FLJ32028 protein, or a fragment thereof. FLJ32028 or a fragment thereof functions as a marker for regulatory T cells because FLJ32028 is expressed specifically on the regulatory T cells. The fragment of FLJ32028 is preferably a peptide part thereof outside the cell membrane. The marker of the present invention can be used for detecting regulatory T cells.

Method for Detecting Regulatory T Cells

Regulatory T cells can be detected in subject cells by allowing the subject cells on the surface of which the transmembrane molecule FLJ32028 protein is suspected to be expressed to contact with the antibody of the present invention to detect the expression of the transmembrane molecule FLJ32028 protein. For example, a fluorescent dye can be attached to a monoclonal antibody capable of specifically binding to FLJ32028, which is then mixed with a peripheral blood of a cancer patient to allow the monoclonal antibody to bind to the transmembrane molecule FLJ32028, followed by measurement using a flow cytometer or the like to detect regulatory T cells in the sample. The method for detecting regulatory T cells according to the present invention can provide an indication for determining the amount of the monoclonal antibody necessary for treatment, for example, by measuring the marker in the patient' blood before administering the medicament of the present invention to detect regulatory T cells in the combined use of the medicament of the present invention and the immuno-cell therapy.

EXAMPLES

The present invention is described below in detail with reference to Examples. However, it is to be understood that the invention is not intended to be limited thereto.

Example 1

Identification of FLJ32028 as Marker for Induced Treg (iTreg)

To identify a specific molecule expressed on iTreg surface, cells were prepared as follows, followed by identifying the molecule by a gene subtraction method.

<Preparation of Cells>

First, each Treg, Th1 and Th2 for use in the experiment were prepared.
The medium used for culture was a complete medium when Th1 and Th2 cells were induced.

The composition of the complete medium was as follows.
RPMI1640 (from Invitrogen) including 10% FBS (from Equitech-Bio, Inc)
25mM HEPES (from Invitrogen)
$5\times10^{-5}$ M 2-mercaptoethanol (from Invitrogen)
$2\times10^{-5}$ M L-glutamine (from Invitrogen)
$1\times10^{-5}$ M sodium pyruvate (from Invitrogen)
1% non-essential amino acids (from Invitrogen)
100 U/ml penicillin/100 mg/ml streptomycin (from Invitrogen)

Peripheral blood mononuclear cells (PBMC) were isolated from healthy donor whole blood by piling up the whole blood onto Lympho prep (from Axis-Shield Poc AS), followed by centrifugation (centrifuge: 430 g, 30 min., RT (centrifugal machine: KUBOTA5910).

Next, $CD^{4+}$ T cells and $CD^{4-}$ T cells were prepared using the human CD4 multisort kit (from Miltenyi Biotec GmbH).
PBMC is first reacted with CD4 microbeads and divided into a $CD4^+$ fraction and a $CD4^-$ fraction. The $CD4^+$ fraction was reacted with CD45RA beads or CD25 beads to provide a $CD4^+CD45RA^+$ fraction or a $CD4^+CD25^+$ fraction.
That is, $CD4^+CD45RA^+$ (naive T cells) and $CD4^+CD25^+$ were obtained from the positive fraction and $CD4^+CD45RA^-$ (memory T cells) and $CD4^+CD25^-$ were obtained from the negative fraction.

Here, $CD4^+CD45RA^+$ are naive T cells undergoing no antigenic stimulation and induced to Th1 or Th2 by reaction with antigen-presenting cells (APC) as described later.
Then, the $CD4^+CD25^+$ fraction is used as Treg, and dissolved in Isogen reagent after confirming the expression thereof using a flow cytometer (to RNA preparation).

The $CD4^+CD45RA^+$ fraction was subjected to induction from the naive T cells to Th1 or Th2.
In the induction of Th1 or Th2, cells used as APC were those obtained by adding 25 μg/ml of mitomycin (from Wako Pure Chemical Industries Ltd.) to the $CD4^-$ fraction and reacting the mixture at 37°C for 30 minutes, followed by washing 4 times with the culture medium.

APC thus obtained were used to induce Th1 or Th2.
Naive T cells ($CD4^{1-}CD45RA^+$) were cultured under Th1-inducing conditions or under Th2-inducing conditions to induce effectors, Th1 or Th2.

Th1: $CD4^+CD45RA^+$ T cells ($1\times0^6$) + APCs ($2\times10^6$) + plate coated 5 μg/ml CD3 antibody (2C11; from BD Pharmingen) + 5 μg/ml CD28 antibody (CD28.2; from BD Pharmingen) + 5 μg/ml anti-IL-4 antibody (MP4-25D2; from BD Pharmingen) + 2.5 ng/ml rIL-12 (from PeproTeck EC Ltd.) + 10 U/ml rIL-2 (from BD Pharmingen).
The above Th1 was induced referring to J Immunol. 2002 Aug 5; 169 (4): 1893-903.

Th2: $CD4^+CD45RA^+$ T cells ($1\times10^6$) + APCs ($2\times10^6$) + 1 mg/ml thalidomide (from Sigma-Aldrich) + 10 μg/ml PHA (from Sigma-Aldrich) + 2 ng/ml IL-12 antibody (C8.6, from BD Pharmingen) + 20 ng/ml rIL-4 (from BD Pharmingen) + 10 Uml rIL-2
Th2 was induced referring to Clin Exp Immunol. 1995 Feb; 99 (2): 160-7.

<Confirmation of Th1 and 2>

Then, the induced Th1 or Th2 cells were subjected to mitogen stimulation using PMA and ionomycin, and were confirmed to be desired cells by intracellularly staining produced IFN-γ characteristic of Th1 and produced IL-4 characteristic of Th2.

PMA (from Sigma-Aldrich) (40 ng/ml) and ionomycin (from Sigma-Aldrich) (4 ng/ml) were first added to Th1 or Th2 cells, which was then cultured for 4 hours. Two hours before the end of reaction, 2 μM Brefeldin A (from Sigma-Aldrich) was added.

After reaction, the cells were recovered and fixed and subjected to membrane permeation treatment using IntraPrep (from Beckman Coulter).

The staining was then carried out using human PE-IFN-γ antibody (45.15, from Beckman Coulter) and human FITC-IL-4 antibody (4D9, from Beckman Coulter).
Thereafter, the production of IFN-γ in Th1 cells and the production of IL-4 in Th2 cells were confirmed by measurement using Epics XL (from Beckman Coulter).

<Subtraction>

<u>Reagents Used for RNA Preparation, cDNA Synthesis, and Gene Subtraction</u>

First, RNAs of the cells ($CD4^+CD25^+$ T cells, $CD4^+CD25^-$ T cells, Th1, and Th2) used for the subtraction were prepared according to the following protocol using Cell suspended with Isogen TRIzol (from Wako Pure Chemical Industries Ltd.).

The cells were suspended in Isogen, and chloroform was added to the suspension, which was then vortexed, followed by centrifugation under conditions of 14,000 rpm, 15 minutes and 4°C.
Subsequently, 2-propanol was added to the centrifuged supernatant, which was then centrifuged under conditions of 14,000 rpm, 10 minutes and 4°C. Thereafter, 70% ethanol was added to the pellet, which was then centrifuged at 14,000 rpm for 15 minutes at 4°C, followed by dissolving the pellet in DEPC water.

cDNA was then synthesized according to the following protocol, using the SMARTTM PCR cDNA synthesis kit instruction (from BD Bioscience).
CDS primer was hybridized to 3' to each of the RNAs obtained from the respective cells and allowed to bind to cDNA using a reverse transcriptase. Long distance PCR was then carried out using 5' PCR primer IIA and Advantage polymerase, followed by recovering a PCR product to provide cDNA.

To identify a transmembrane molecule specific to $CD4^+CD25^+$ T cells, the described subtraction was then performed.
When formulated, the outline is as follows.

$$(CD4^+CD25^+ \text{ T cells}) - (CD4^+CD25^- \text{ T cells, Th1, Th2})$$

The subtraction was carried out using a Clonetech PCR-Select(TM) cDNA subtraction kit instruction (from BD Bioscience Clonetech). The protocol was performed according to the user manual included in the PCR-Select(TM) cDNA subtraction kit to provide a gene product dominantly expressed on Treg.

The gene product obtained by the subtraction was ligated to the pGEM-T Easy Vector as a vector for subcloning and sequenced.

<Cloning>

The PCR product by the subtraction was ligated to pGEM-T EASY vector (from Promega Corporation) and transformed into E. coli DH5α (from Toyobo Co., Ltd.). The DH5α was seeded on an AMP (ampicillin, from Meiji Seika Kaisha, Ltd.), X-gal (from Sigma-Aldrich) and IPTG (from Sigma-Aldrich) selection LB plate. After culture at 37°C overnight, white colonies of the colonies generated on the plate were selected and subjected to colony PCR using T7 primer and SP6 primer to amplify the insert fragment.

The amplified fragment as the above PCR product was purified with Microcon-PCR (from Millipore), and the purified product was used as a template to perform a sequencing reaction employing T7 primer and SP6 primer. Three hundred clones of the PCR product were obtained, sequenced using a DNA sequencer, and subjected to the identification of gene names by BLAST search.

Of the 300 clones obtained by the subtraction, 30 clones were selected as clones predicted to be membrane proteins by BLAST search or a Website predicting protein structures (for example, SOSUI or Human Protein Reference Database). These 30 clones were subjected to real-time PCR and examined for molecules dominantly expressed at a mRNA level in regulatory T cells; as a result, 8 clones were selected. Fig. 1 shows the results of the real-time PCR of FLJ32028 as one of the clones. FLJ32028 was dominantly expressed in Treg compared to in $CD4^+CD25^-$ T cells, Th1 cells, and Th2 cells; thus, it has been researched as a marker for Treg.

Cloning of FLJ32028 Gene

The cDNA fragment prepared from the RNA extracted from healthy donor peripheral blood lymphocytes was used as a template to amplify a human FLJ32028 gene fragment (the full length of gene between the start codon and the stop codon) by a PCR method. Primer sequences were used by synthesizing the following two DNA oligomers. The underlined portions in the following primer base sequences mean restriction enzyme recognition-sites *Hin* dIII (FLJ32028 F) and *Xba* I (FLJ32028 R) for insertion of a vector.
Primer FLJ32028 F: 5'-AATAAGCTTGCCACCATGCAGGCTCCCCGCGCAGCCCTAGTCTTCGCCCTGGTG-3'
Primer FLJ32028 R: 5'-CCATCTAGATTAGGATTCACTGTCACTTGGGTTGTGATTTG-3'
Using KOD plus DNA polymerase (from Toyobo Co., Ltd.), the PCR was carried out under reaction conditions in which 94°C for 15 seconds, 55°C for 30 seconds, and 68°C for 30 seconds formed one cycle and the cycle was repeated 35 times, after placing the reaction at 94°C for 2 minutes. The DNA fragment specifically amplified by the PCR method was separated by 1.5% agarose gel electrophoresis, cut out, and treated with the restrictive enzymes *Hin* dIII and *Xba* I. Similarly, a plasmid vector, pRc/CMV (from Invitrogen), was treated with the restriction enzymes, and the DNA fragment was inserted into the *Hin* dIII and *Xba* I sites to provide pRc/CMV-FLJ32028.

Example 2

Preparation of Monoclonal Antibody to FLJ32028: Preparation of Hybridoma

The FLJ32028 gene fragment (the full length of sequence between the start codon and the stop codon: the gene between nucleotides 1 and 552 in SEQ ID NO: 1 of the Sequence Listing) is incorporated in a tagged mammalian expression vector. It was verified using hamster-derived CHO cells before immunization whether or not the gene construct obtained was expressed on the cell surface as designed. That is, the gene construct obtained was transiently expressed and introduced into the CHO cells.

The CHO cells having the construct introduced were cultured in a $CO_2$ incubator for 24 hours and used for flow cytometry (FCM) analysis.

In performing the FCM analysis, Myc was added as an antibody to the tag added to the introduced gene to a cell suspension containing the cultured gene-introduced cells, which was then allowed to stand for 30 minutes. Thereafter, a fluorescently labeled secondary antibody specifically recognizing the tag was added to the cell suspension, which was then used for FCM analysis after 30 minutes of standing. We confirmed that the gene construct made in the present invention was expressed on the cell surface.

The gene construct was then injected into six 6- to 8-week old female Balb/c mice using a gene gun, and the mice were breeded for about 2 to 3 months.

Thereafter, the above CHO cells having the introduced human FLJ32028 gene were used for the analysis of the serum collected from the immunized animals, i.e., the analysis of a polyclonal antibody.

That is, the tagged w8/ FLJ32028 construct was transiently expressed and introduced into CHO cells, and the CHO cells having the construct introduced were cultured in a $CO_2$ incubator for 24 hours and used for FCM analysis.

In performing the FCM analysis, the serum (polyclonal antibody) collected from the animal immunized with the introduced gene was added to a culture medium containing the cultured w8/ FLJ32028-introduced cells, which was then allowed to stand for 30 minutes. Thereafter, a fluorescently labeled secondary antibody specifically recognizing the immunoglobulin from the immunized animal was added to the medium, which was then used for FCM analysis after 30 minutes of standing.

The animal producing a strong specific antibody recognizing the human FLJ32028 gene-introduced cells was dissected to take out spleen cells, and the spleen cells were fused with SP2/0 mouse myeloma cells in amounts of $4\times10^7$ cells and $1\times10^8$ cells, respectively, using polyethylene glycol. Seven to ten days after fusion, using flow cytometry (FCM) as a means for selecting hybridoma cell lines producing monoclonal antibodies recognizing the tertiary structure of human FLJ32028 from among the hybridoma cell lines, human FLJ32028 gene-introduced CHO cells were used to transiently express and introduce (transfect) the vv8/ FLJ32028 construct into CHO cells. The mammalian cells having the construct introduced were cultured in a $CO_2$ incubator for 24 hours and used for FCM analysis.

In performing the FCM analysis, a part of a culture supernatant of each hybridoma was added to a culture medium containing the cultured vv8/ FLJ32028-introduced cells, which was then allowed to stand for 30 minutes. Thereafter, a fluorescently labeled secondary antibody specifically recognizing a mouse immunoglobulin was added to the medium, which was then used for FCM analysis after 30 minutes of standing.

As a result, one clone of hybridoma producing anti-FLJ32028 antibody was obtained. This hybridoma was designated as Mouse-Mouse hybridoma FLJ32028-41 and deposited in International Patent Organism Depositary, National Institute of Advanced Industrial Science and Technology (Higashi 1-chome 1-banchi 1 chuo dai-6, Tsukuba City, Ibaraki, Japan) March 6, 2008 (accession number: FERM P-21522). The deposit was transferred to the international deposit according to the Budapest Treaty February 26, 2009 (accession number: FERM ABP-11100).

For specific antibody mass production in the hybridoma producing anti-FLJ32028 antibody, $1\times10^6$ to $1\times10^7$ cells/mouse of established hybridoma cells were injected into abdominal cavities of nude mice. Ascites fluid accumulates in the abdominal cavity of the nude mice owing to the proliferation of the hybridoma. The ascites fluid was collected, and a high-purity immunoglobulin was purified from the ascites fluid using a column filled up with resin for purification of Protein G Sepharose (from GE). Subsequent analysis was carried out using this purified monoclonal antibody.

Example 3

Confirmation of Antibody Verification That This Antibody Recognizes FLJ32028

Using the gene encoding FLJ32028 obtained in Example 2, $5\times10^5$ CHO cells were transfected with 4 $\mu$g of an expression vector obtained by incorporating the gene encoding the FLJ32028 molecule in a plasmid (pRC-cmv vector, from Invitrogen) (pRC-FLJ32028). Specifically, 4 $\mu$g of the DNA plasmid was mixed with 10 $\mu$l of lipofectoamine 2000 (from Invitrogen), which was then added to CHO cells. Four hours later, the culture medium was exchanged, and 24 to 48 hours later, the CHO cells were recovered and subjected to FACS analysis.

The above transfectants were reacted at 4°C with 100 μl of the FLJ32028 hybridoma supernatant obtained in Example 2 bound to 2 μl of an anti-mouse IgG1, and the reaction was analyzed by FACTS. For comparison, a transfectant obtained by incorporating a DNA plasmid encoding a protein independent of FLJ32028 (mock) in CHO cells were reacted with the above supernatant + anti-mouse IgG1, and the reaction was also analyzed by FACS. The results are shown in Fig. 2.

Fig. 2 is a graph of FACS analysis showing the reaction of the FLJ32028 transfectant and the mock transfectant with the anti-FLJ32028 antibody. Fig. 2 shows that the FLJ32028 transfectant is reacted with the supernatant obtained in the above Example. This demonstrated that the antibody was an antibody recognizing FLJ32028.

Example 4

<u>Depletion of Treg Using Antibody and Effect of Depletion (Depletion Effect of Antibody Magnet Bead)</u>

To examine the Treg-depleting effect of the antibody of the present invention, cell culture was carried out by depleting Treg in the blood using the antibody of the present invention, and the degree of cell proliferation was measured.

<Preparation of Cells>

As described in the above Example, the peripheral blood mononuclear cells used for the test were obtained by collecting 30 to 50 ml of blood in heparin from a healthy donor, diluting the whole blood in an equal amount of PMI1640 medium, and piling up the diluted solution on Lymphoprep, followed by centrifugation at 1,500 rpm at room temperature.

<Preparation of Antibody Bead>

Using epoxy group-activated beads M450, and tosyl group-activated beads M450 (Dynalbeads M450 Epoxy and Dynalbeads M450 Tosyl, from Veritas) as magnetic beads, the following 6 types of antibody binding beads were prepared as beads binding to the antibody of the present invention and an antibody for comparison. The reason why the two types: Epoxy and Tosyl were used is that magnetic beads efficiently bound to an antibody vary depending on the type of the antibody.

Epoxy group-activated control IgG1
Tosyl group-activated control IgG1
Epoxy group-activated anti-CD25 antibody (IgG1)
Tosyl group-activated anti-CD25 antibody (IgG1)
Epoxy group-activated anti-FLJ32028 antibody (IgG1)
Tosyl group-activated anti-FLJ32028 antibody (IgG1)

A method for preparing the antibody binding beads involved mixing $2\times10^7$ beads, control, 50 μg of anti-CD25 or anti-FLJ32028 antibody under conditions of 37°C and 2 hours to provide bound beads.

<Analysis of Treg-Depleting Function>

The beads prepared as described above were used to perform analysis as follows.
Healthy donor PBMC (30 to 50 ml) obtained by the above blood collection and separation were cultured in a 75-$cm^2$ flask (from Sumiron) under conditions of 37°C and 2 hours, and adhering cells such as mainly monocytes were removed. PBMC pretreated described above were divided into a group for non-depletion and groups for co-culture with the above 6 types of beads in amounts of 1 ml for each group, and 50 μl of beads were mixed with the respective groups of PBMC, followed by incubating each mixture on a shaker for 30 minutes in a refrigerator at 4°C.

Subsequently, cells adhering to a magnetic bead-antibody complex were removed using a magnet for Dynal' exclusive use from each test tube; the remaining PBMC were stimulated with an anti-CD3 antibody to perform cell proliferation measurement using a tetrazolium salt (WST-1).
WST-1 is decomposed into a formazan dye by mitochondrial succinate-tetrazolium reductase active only in live cells; thus, the WST-1 measurement involves measuring cell proliferation by the dye change.

WST-1 (10 μl) was added to each group of PBMC (200 μl), having been subjected to the anti-CD3 stimulation for 2 to 3 days after the bead removal, followed by measuring absorbance at 450 nm after 30 minutes of standing and 90 minutes of standing.
The results of the measurement are shown in Fig. 3. As shown in Fig. 3, the depletion of Treg using FLJ32028 antibody enhanced cell proliferation compared to the non-treatment and control.

<Cytotoxic Activity Test>

PBMC after bead operation were used to analyze cytotoxic activity against cancer cells and the amount of intracellular IFN-γ production.
For the cytotoxic activity, a cancer cell line, K562, was first used as a target and mixed with cultured cells in a target : cultured cells ratio of 1 : 1 to examine their cytotoxic activity.
K562 cell line (purchased from ATTC) was cultured with DMEM medium (from GIBCO) and subcultured every 3 to 4 days.
These cells were recovered and then suspended in RPMI1640 medium (from GIBCO), to which 1 μl of Calcein-AM (from DOJINDO, 10 mg/ml) was added, followed by allowing the mixture to stand at 37°C for 40 minutes. Thereafter, the mixture was mixed with PBMC as effector cells at a ratio of 1:1 1 (96-well plate (from Sumiron), final liquid volume: 150 μl, $2\times10^4$ cells), which was then reacted at 37°C in the presence of 5% $CO_2$ for 4 hours. Four hours later, cytotoxic activity was measured using TeraScan system.
For the proportion of intracellular IFN-γ, PBMC in each group were recovered 3 days after culture and re-stimulated with PMA (40 ng/ml, from Sigma) and ionomycin (4 μg/ml, from Sigma) for 4 hours. CD4 or CD8 positive cells in these cells were subjected to FACS analysis of IFN-γ production.
These measurement results are shown in Figs. 4 and 5.

Fig. 4 is a graph showing cytotoxic activity in each cell group after bead removal.
As shown in the figure, the cytotoxic activity was highest in the group of treatment with beads bound to the antibody of the present invention.
Fig. 5 is a graph showing the proportion of IFN-γ production in each cell group.
Also in this figure, the production amount was largest in the group of treatment with beads bound to the antibody of the present invention.

As described above, the lymphocytes treated (Treg-depleted) with the antibody of the present invention showed an enhanced proliferation rate and further functional improvement.

Example 5

Effect of Antibody When Added to Culture Medium to Culture Cells

It was then examined to what extent the continuous presence of the antibody of the present invention during cell proliferation depletes Treg as a factor responsible for cell inhibition and has the effect of enhancing cell proliferation.

<Examination of Immobilized FLJ32028 Antibody Etc.>

The antibodies used for this experiment were first immobilized in a culture vessel.
The anti-FLJ32028 antibody and IgG1 as a negative control were used.
An anti-mouse goat IgG polyclonal antibody (Sigma) (5 μg/ml (PBS)) was first placed in a 96-well plate (from Sumiron), allowed to stand at 37°C for 2 hours, and thereby immobilized. (This step enables to get clear results because it can increase the titer of an antibody for testing to be next immobilized.)
Subsequently, 5 μg/ml each of anti-CD3 antibody (also known as Orthoclone, from Janssen Pharmaceutical K.K.), anti-FLJ32028 antibody, control IgG1 and anti-GITR antibody were individually placed in the plate in which the above polyclonal antibody was immobilized, and immobilized (at 37°C for 2 hours in a $CO_2$ incubator).
PBMC ($3\times10^4$ cells) obtained from a healthy donor (in the same way as in the above Example) were seeded in each antibody-immobilized vessel and cultured at 37°C in a $CO_2$ incubator for 3 days, to which 10 μl of WST-1 was then added, followed by measuring absorbance after 30 minutes of standing and 90 minutes of standing.

< Examination of FLJ32028 Antibody ETC. When Added to Culture Medium>

As in the above immobilization test, the anti-mouse goat IgG polyclonal antibody was preliminarily immobilized in a vessel.
Five μg/ml each of anti-CD3 antibody, anti-FLJ32028 antibody, and control IgG1 were placed in the vessel having the polyclonal antibody immobilized simultaneously with the addition of the 10% FCS RPMI1640 medium, to which $2\times10^4$ PBMC and WST-1 were further added as in the above immobilization test, followed by measuring absorbance after 30 minutes of standing and 90 minutes of standing.
The results of the above examinations are shown in Fig. 6.
As shown in Fig. 6, the antibody of the present invention (anti-FLJ32028 antibody) promoted the degree of cell proliferation, i.e., canceled the suppression of cell proliferation compared to the control antibody in both cases of immobilization and continuous addition.

Example 6

Selection of iTreg (Selection by Staining)

Then, in order to observe the effect of the antibody of the present invention on the induced Treg (iTreg) believed to occur particularly around tumor tissue, iTreg were first obtained.

Anti-CD3 antibody (Orthoclone, 5 μg/ml) and anti-CD28 antibody (from BD Pharmingen, 5 μg/ml) were immobilized in a 24-well plate (from Sumiron). Then, as described in the above Example, blood was collected from a healthy donor and a $CD4^+CD25^-$ fraction was separated therefrom using MACS beads (depletion of nTreg ($CD4^+CD25^+$ fraction cells)).

T cells ($CD4^+CD25^-$ T cells) ($5\times10^5$ cells) of this fraction were seeded on the 24well plate subjected to immobilization treatment and cultured.

The composition of the culture medium used was rIL-2 (60 U/ml), rIL-9 (42 U/ml), rIL-15 (1 ng/ml), rTGF-β1 (32 U/ml), anti-IL-12 antibody (20 μg/ml), and anti-IFN-γ antibody (10 μg/ml)) added to 10% FCS RMPI1640 medium. After 3 days of culture, cells were separated from the above antibody-immobilized plate and further cultured for 3 to 4 days using the same medium and vessel.

Six to seven days after the start of culture in the above medium, cells were recovered; the surface of the cultured cells was stained with anti-FLJ32028 antibody; and the cells were fixed with PBS containing 1% paraformaldehyde, treated with a surfactant comprising saponin, reacted with 10 μL of anti-FoxP3 antibody at 4°C for 30 minutes, and then analyzed by FACS.

The analysis results are shown in Fig. 7. As shown in the figure, the expression of FLJ32028 was observed in the fraction positive for FoxP3 considered to be most useful as a marker for Treg. From this result, cells of this FLJ32028-positive fraction was judged to be iTreg and selected and used in subsequent studies.

Example 7

Effect of Culture When Culture Was Performed Using Resultant iTreg and Antibody of Present Invention

Mixed lymphocyte reaction (MLR) employing allo-lymphocytes was carried out using the iTreg selected in Example 6 above and anti-FLJ32028 antibody as the antibody of the present invention.

The lymphocytes used for the mixed lymphocyte reaction were obtained from two healthy donors having different HLA types. Lymphocytes (called donor A lymphocytes) were collected from a healthy donor identical to the donor whose lymphocytes were induced into iTreg and labeled with CFSE. CFSE has the property of having a fluorescent intensity decreasing in half after each cell proliferation. The CFSE-labeled lymphocytes were mixed with lymphocytes having a different HLA type (called donor B lymphocytes) treated with MMC (mitomycin) (30 μg/ml, 37°C, 30 minutes) in a ratio of donor A lymphocytes : donor B lymphocytes of 1 : 1 (MLR).

In this allo-MLR, iTreg were mixed to a ratio of iTreg : donor A lymphocytes of 1 : 1 or 1 : 10; cells were seeded at a final cell number of $2\times10^5$ on a 48-well plate (from Sumiron) to perform a Treg-suppressing assay. Here, 2 μg/ml of control IgG1 antibody or anti-FLJ32028 antibody was added; 10% FCS-RPMI1640 medium was used as a culture medium; and the culture was carried out at 37°C for 4 days.

Four days after culture, the cultured cells were recovered, and the CFSE-labeled lymphocytes were subjected to FACS analysis for the division frequency thereof.

The results of this test are shown in Fig. 8. As shown in the figure, the culture by the addition of the antibody of the present invention caused the proliferation of donor A lymphocytes to be recovered, although the proliferation thereof remained suppressed in the control.

Example 8

Effect of Culture by Addition of Anti-FLJ32028 Antibody in Inducing iTreg on Expression of FoxP3 and Its Suppressive Effect

Blood was collected from each of the healthy donors, and $CD4^+CD25^-$ T cells were obtained using MACS beads as described in Example 6. Using the culture medium described in Example 6 (RPMI1640, 10% FCS, rIL-2, rIL-9, rIL-15, rTGF-β1, anti-IL-12 antibody, anti-IFN-γ antibody), $1\times10^6$ $CD4^+CD25^-$ T cells were cultured to induce iTreg. Here, the culture was carried out under each of the 4 conditions consisting of using an antibody so that (1) control IgG1

(50 μg/ml), (2) anti-FLJ32028 antibody (1 μg/ml), (3) anti-FLJ32028 antibody (10 μ/ml), or (4) anti-FLJ32028 antibody (50 μg/ml) is added. Seven days later, cells were recovered, and the expression of CD25/FoxP3 was measured using a flow cytometer. Using CD3-LAK cells induced from PBMC obtained from an identical healthy donor employing anti-CD3 antibody and rIL-2 and PBMC co-cultured with U937 as the respective effector cells, each effector cells and U937 as target cells were co-cultured in a ratio of effector cells : U937 of 1 : 1 or 10 : 1 to measure cytotoxic activity. Here, the Treg obtained under each of the conditions of (1) to (4) in the above culture were added to a ratio of Treg / effector cells of 1/10 to measure how cells obtained under conditions of (1) to (4) influence the cytotoxic activity of the effector cells.

The respective results are shown in Figs. 9 and 10. When the FoxP3 expression intensity in the control IgG1 addition group is set at 1, the FoxP3 expression intensity in the anti-FLJ32028 antibody addition group was decreased in a manner dependent on the antibody concentration (Fig. 9). This demonstrated that the antibody of the present invention had the effect of suppressing the differentiation of $CD4^+CD25^-$ T cells into iTreg. The iTreg cultured by addition of control IgG1 suppressed the cytotoxic activity of PBMC, while the iTreg cultured by addition of anti-FLJ32028 antibody had the suppression of the cytotoxic activity canceled and the canceling effect was dependent on the concentration of anti-FLJ32028 antibody. This demonstrated that the antibody of the present invention can suppress the differentiation of iTreg to cancel the effect of suppressing immune.

Example 9

Effect of Addition of Anti-FLJ32028 Antibody after Inducing itreg on Cytotoxic Activity

As described in Example 6, iTreg were induced from healthy donor's $CD4^+CD25^-$ T cells. The culture was then carried out for 2 days under each of the 4 conditions consisting of using an antibody so that (1) control IgG1 (50 μg/ml), (2) anti-FLJ32028 antibody (1 μg/ml), (3) anti-FLJ32028 antibody (10 μg/ml), or (4) anti-FLJ32028 antibody (50 μg/ml) is added. CD3-LAK were induced from PBMC of an identical healthy donor, and the CD3-LAK and U937 as target cells were co-cultured in a ratio of CD3-LAK : U937 of 1 : 1 or 10 : 1 to measure cytotoxic activity. Here, iTreg obtained by the culture under each of the 4 conditions were added in a ratio ofTreg / CD3-LAK of 1/10. The results are shown in Fig. 11. No difference was observed between all groups, suggesting that the addition of anti-FLJ32028 antibody after inducing iTreg did not affect the ability of iTreg to suppress immune.

Example 10

Effect of Anti-FLJ32028 Antibody on Anti-Tumor Immunity in Immuno-deficient Mice

An immuno-deficient mouse (NOD/SCID) is a mouse in which T and B cells are absent, and NK cells are present, but lose functionality. Human PBMC ($2\times10^7$ cells) derived from a healthy donor were intraperitoneally administered to immuno-deficient mice, and 7 days thereafter, U937 cells ($1\times10^6$ cells) were subcutaneously implanted. Subject mice were divided into the following two groups according to the schedule and amount of intravenous injection of an antibody (5 mice for each group).

(1) A group of intravenous injection of control IgG1 (50 μg/ml) 2, 4, 6, and 8 days after implantation of U937
(2) A group of intravenous injection of anti-FLJ32028 antibody (50 μg/ml) 2, 4, 6, and 8 days after implantation of U937

When the tumor mass of U937 was measured, the tumor mass was most reduced in the (2) group of administration of anti-FLJ32028 antibody (50 μg/ml) 2, 4, 6, and 8 days after. This demonstrated that the antibody of the present invention suppresses a growth of tumor cells when administered into the body.

INDUSTRIAL APPLICABILITY

As described above, the antibody of the present invention has the characteristic of specifically recognizing induced Treg (iTreg). In addition, the antibody of the present invention can inhibit the differentiation of precursor cells of induced regulatory T cells into the induced regulatory T cells. Utilizing such characteristics, the antibody of the present invention can be usefully used in various applications such as efficiently canceling a state of immuno-suppression occurring in a tumor tissue and the like.

## Claims

1. An antibody recognizing a transmembrane molecule, FLJ32028, wherein the antibody specifically binds to regulatory T cells.

2. The antibody according to claim 1, wherein the antibody binds to the transmembrane molecule FLJ32028 specifically expressed on a surface of the regulatory T cells.

3. The antibody according to claim 1 or 2, wherein the regulatory T cells are induced regulatory T cells.

4. The antibody according to any one of claims 1 to 3, wherein the antibody inhibits differentiation of precursor cells of induced regulatory T cell into the induced regulatory T cells.

5. The antibody according to any one of claims 1 to 4, wherein the antibody is a monoclonal antibody.

6. The antibody according to claim 5, wherein the monoclonal antibody is produced from a hybridoma internationally deposited under the Budapest Treaty and given the accession number FERM ABP-11100.

7. A depletion method for regulatory T cells, comprising binding the regulatory T cells to the antibody according to any one of claims 1 to 6 to deplete the regulatory T cells.

8. The depletion method according to claim 7, wherein the regulatory T cells are induced regulatory T cells.

9. A depletion device for regulatory T cells, comprising the antibody according to any one of claims 1 to 6.

10. The depletion device according to claim 9, wherein the regulatory T cells are induced regulatory T cells.

11. A medicament comprising the antibody according to any one of claims 1 to 6.

12. The medicament according to claim 11 for cancelling immuno-suppression due to regulatory T cells.

13. The medicament according to claim 11 or 12 for proliferating cells whose proliferation has been suppressed by regulatory T cells.

14. The medicament according to any one of claims 11 to 13 for enhancing immune function.

15. The medicament according to any one of claims 11 to 14, wherein the regulatory T cells are induced regulatory T cells.

16. A marker for detection of regulatory T cells, comprising a transmembrane molecule, FLJ32028 protein, or a fragment thereof

**17.** The marker for detection of regulatory T cells according to claim 16, wherein the regulatory T cells are induced regulatory T cells.

**18.** A detection method for regulatory T cells in subject cells suspected of expressing a transmembrane molecule, FLJ32028, on the cell surface thereof, comprising contacting the subject cells with the antibody according to any one of claims 1 to 6 to detect expression of the transmembrane molecule FLJ32028 protein on the surface of the subject cells.

**19.** The detection method according to claim 18, wherein the regulatory T cells are induced regulatory T cells.

FIG.1

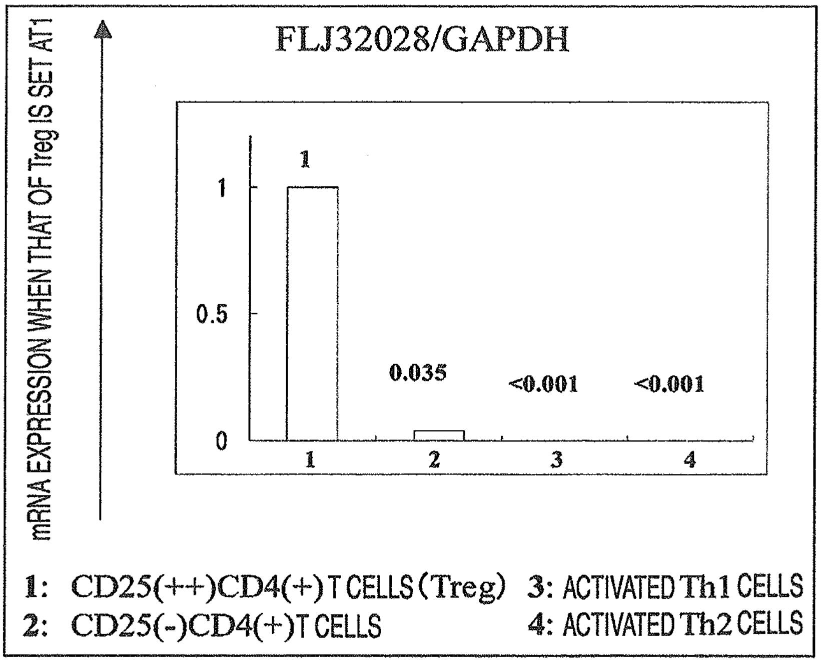
mRNA EXPRESSION WHEN THAT OF Treg IS SET AT1
FLJ32028/GAPDH
1
0.5
0
1
0.035
<0.001
<0.001
1
2
3
4
1: CD25(++)CD4(+)T CELLS(Treg)
2: CD25(-)CD4(+)T CELLS
3: ACTIVATED Th1 CELLS
4: ACTIVATED Th2 CELLS

FIG.2

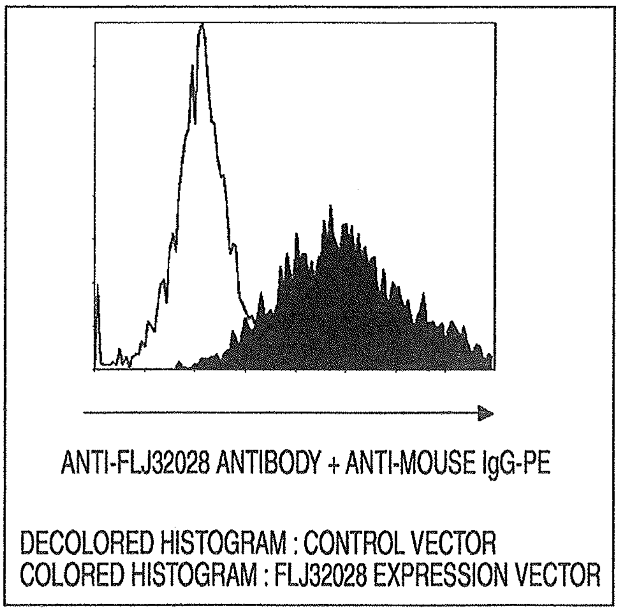
ANTI-FLJ32028 ANTIBODY + ANTI-MOUSE IgG-PE
DECOLORED HISTOGRAM : CONTROL VECTOR
COLORED HISTOGRAM : FLJ32028 EXPRESSION VECTOR

FIG.3

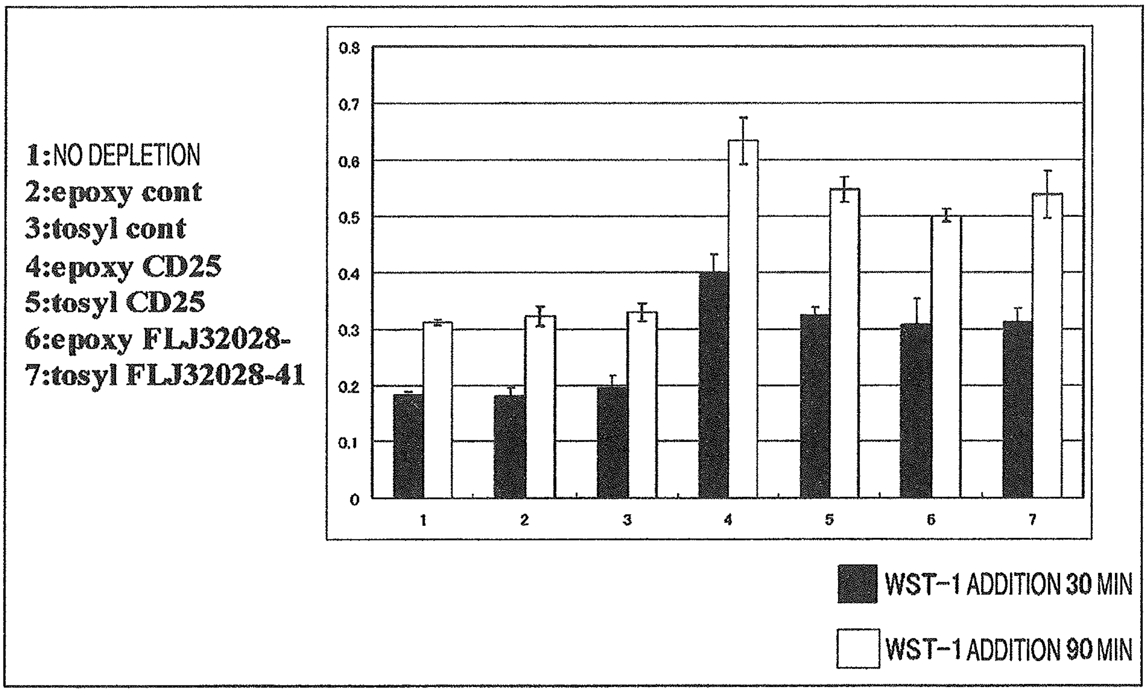
1:NO DEPLETION
2:epoxy cont
3:tosyl cont
4:epoxy CD25
5:tosyl CD25
6:epoxy FLJ32028-
7:tosyl FLJ32028-41
0.8
0.7
0.6
0.5
0.4
0.3
0.2
0.1
0
1
2
3
4
5
6
7
WST-1 ADDITION 30 MIN
WST-1 ADDITION 90 MIN

FIG.4

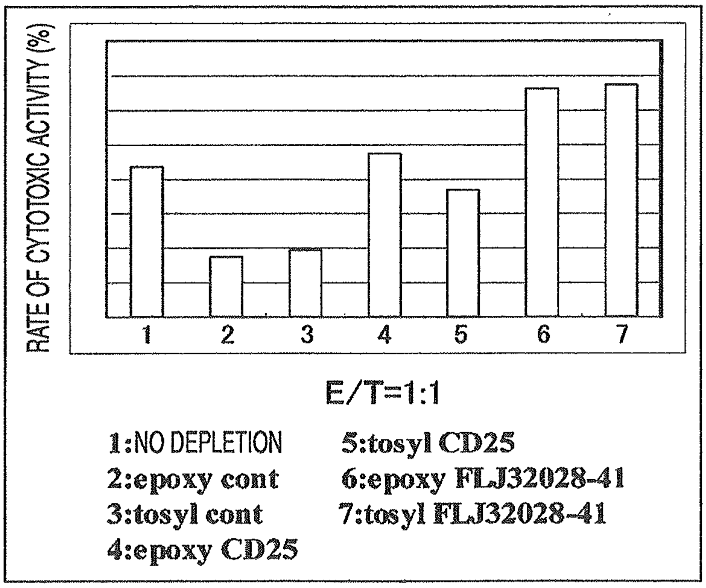
RATE OF CYTOTOXIC ACTIVITY (%)
1
2
3
4
5
6
7
E/T=1:1
1:NO DEPLETION
2:epoxy cont
3:tosyl cont
4:epoxy CD25
5:tosyl CD25
6:epoxy FLJ32028-41
7:tosyl FLJ32028-41

FIG.5

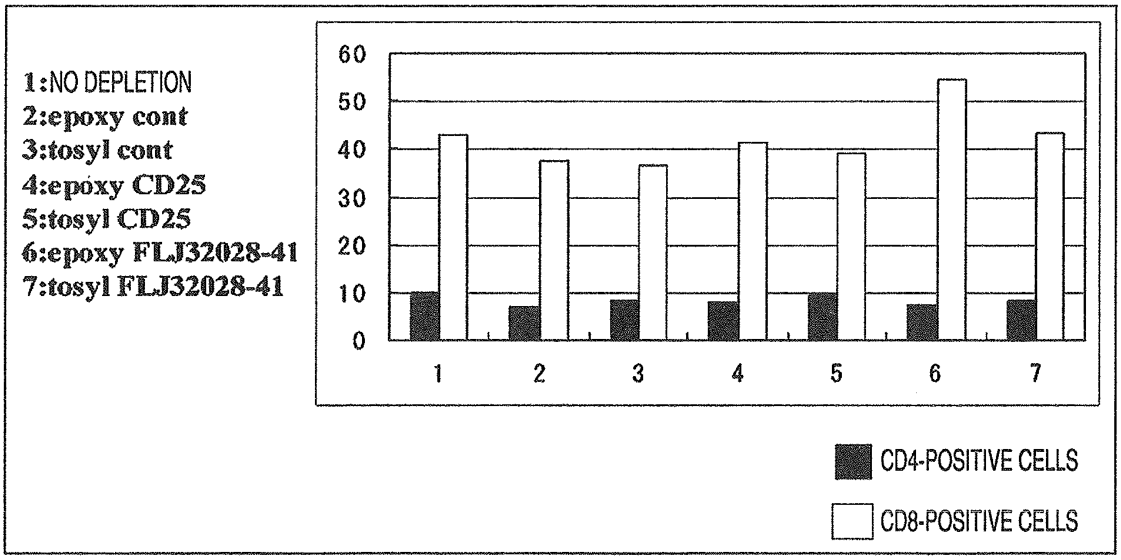
1:NO DEPLETION
2:epoxy cont
3:tosyl cont
4:epoxy CD25
5:tosyl CD25
6:epoxy FLJ32028-41
7:tosyl FLJ32028-41
60
50
40
30
20
10
0
1
2
3
4
5
6
7
CD4-POSITIVE CELLS
CD8-POSITIVE CELLS

FIG.6

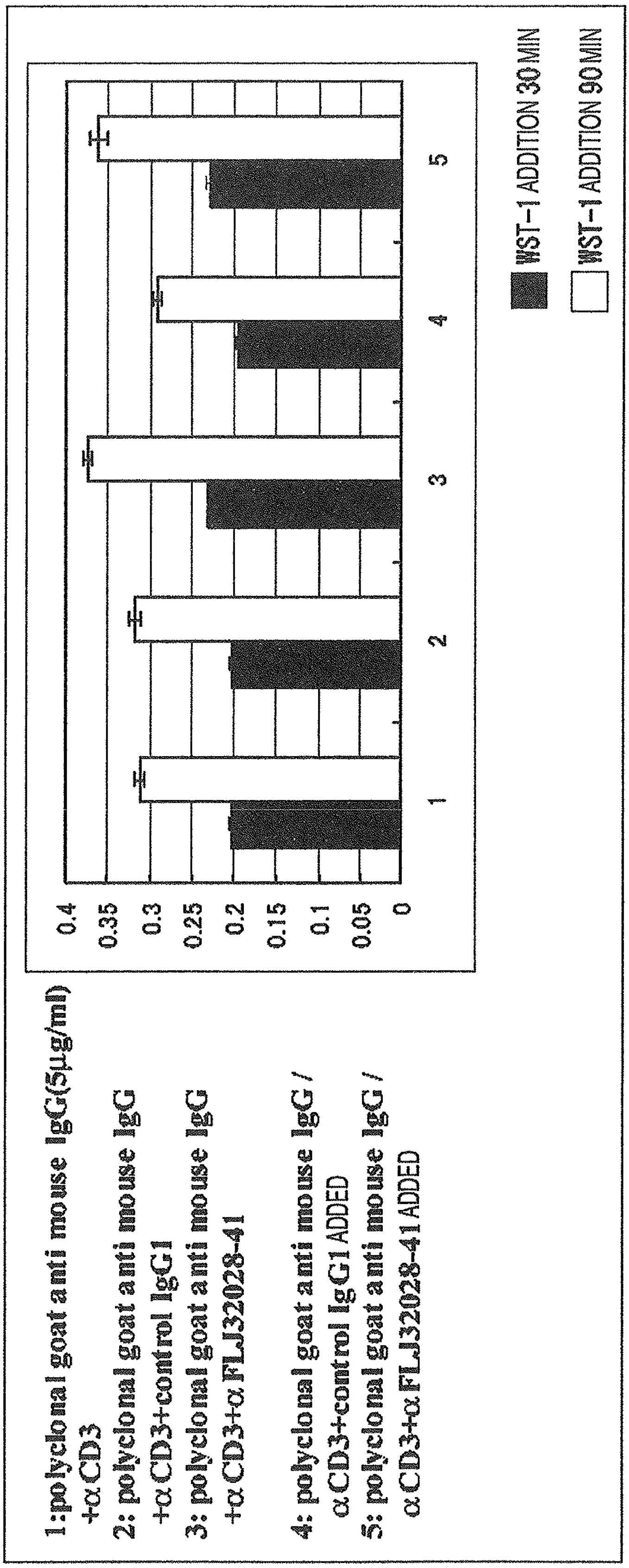
1:polyclonal goat anti mouse IgG(5μg/ml) +α CD3
2: polyclonal goat anti mouse IgG +α CD3+control IgG1
3: polyclonal goat anti mouse IgG +α CD3+α FLJ32028-41
4: polyclonal goat anti mouse IgG / α CD3+control IgG1 ADDED
5: polyclonal goat anti mouse IgG / α CD3+α FLJ32028-41 ADDED
0.4
0.35
0.3
0.25
0.2
0.15
0.1
0.05
0
1
2
3
4
5
WST-1 ADDITION 30 MIN
WST-1 ADDITION 90 MIN

FIG.7

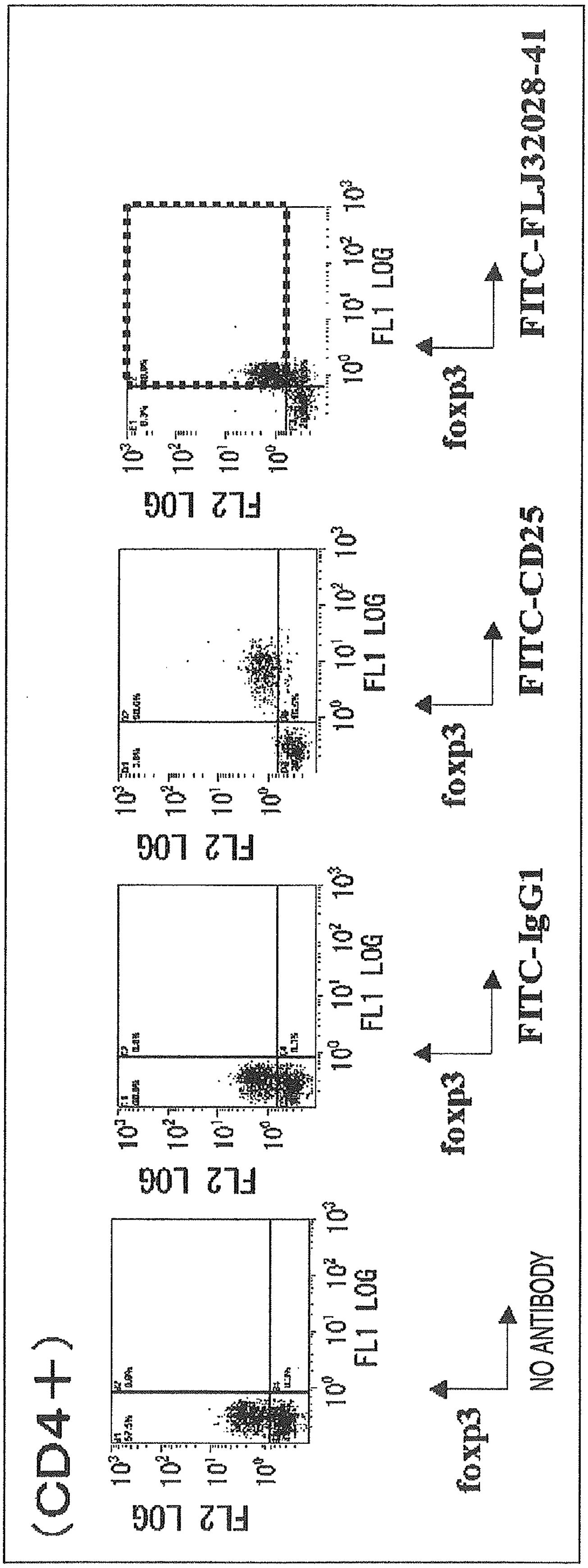
(CD4+)
FL2 LOG
FL1 LOG
foxp3
NO ANTIBODY
FITC-IgG1
FITC-CD25
FITC-FLJ32028-41

FIG.8

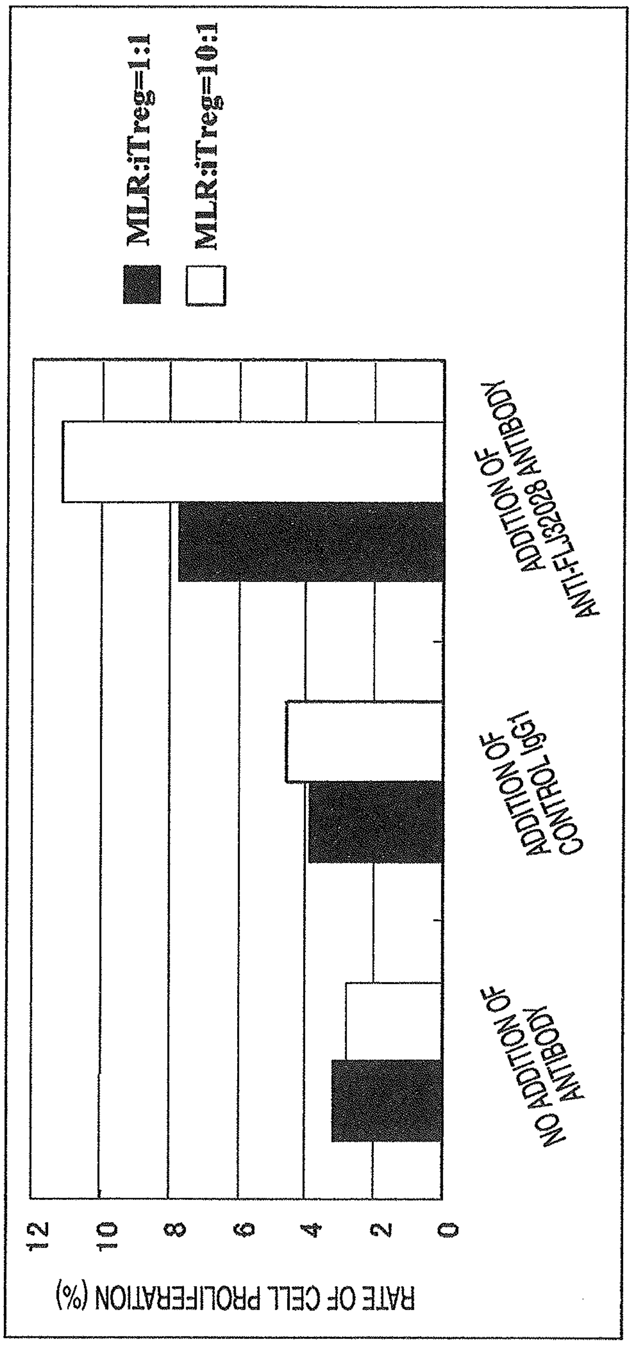
MLR:iTreg=1:1
MLR:iTreg=10:1
RATE OF CELL PROLIFERATION (%)
12
10
8
6
4
2
0
NO ADDITION OF ANTIBODY
ADDITION OF CONTROL IgG1
ADDITION OF ANTI-FLJ32028 ANTIBODY

FIG.9

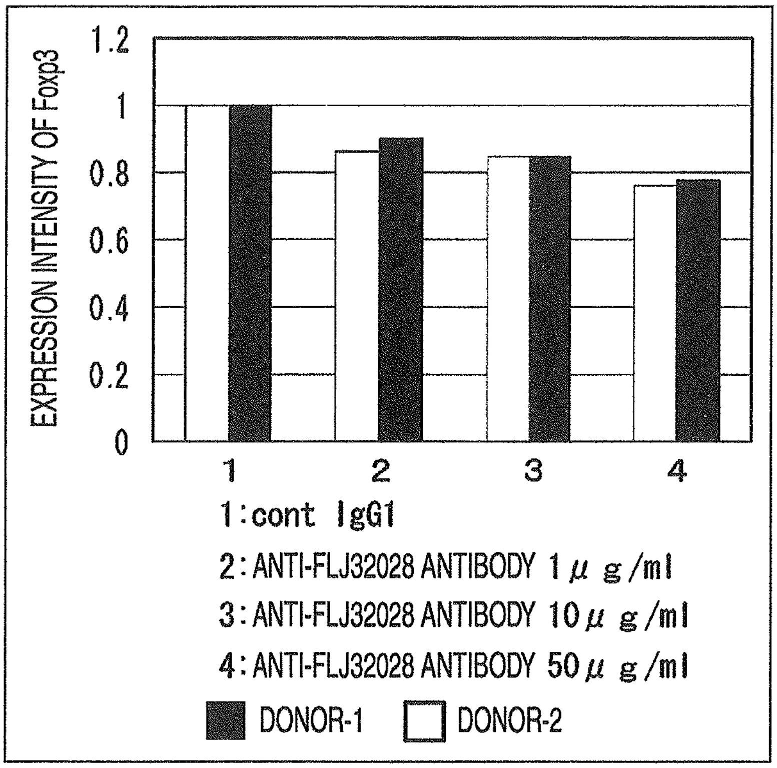
EXPRESSION INTENSITY OF Foxp3
1.2
1
0.8
0.6
0.4
0.2
0
1
2
3
4
1:cont IgG1
2: ANTI-FLJ32028 ANTIBODY 1μg/ml
3: ANTI-FLJ32028 ANTIBODY 10μg/ml
4: ANTI-FLJ32028 ANTIBODY 50μg/ml
DONOR-1
DONOR-2

# FIG.10

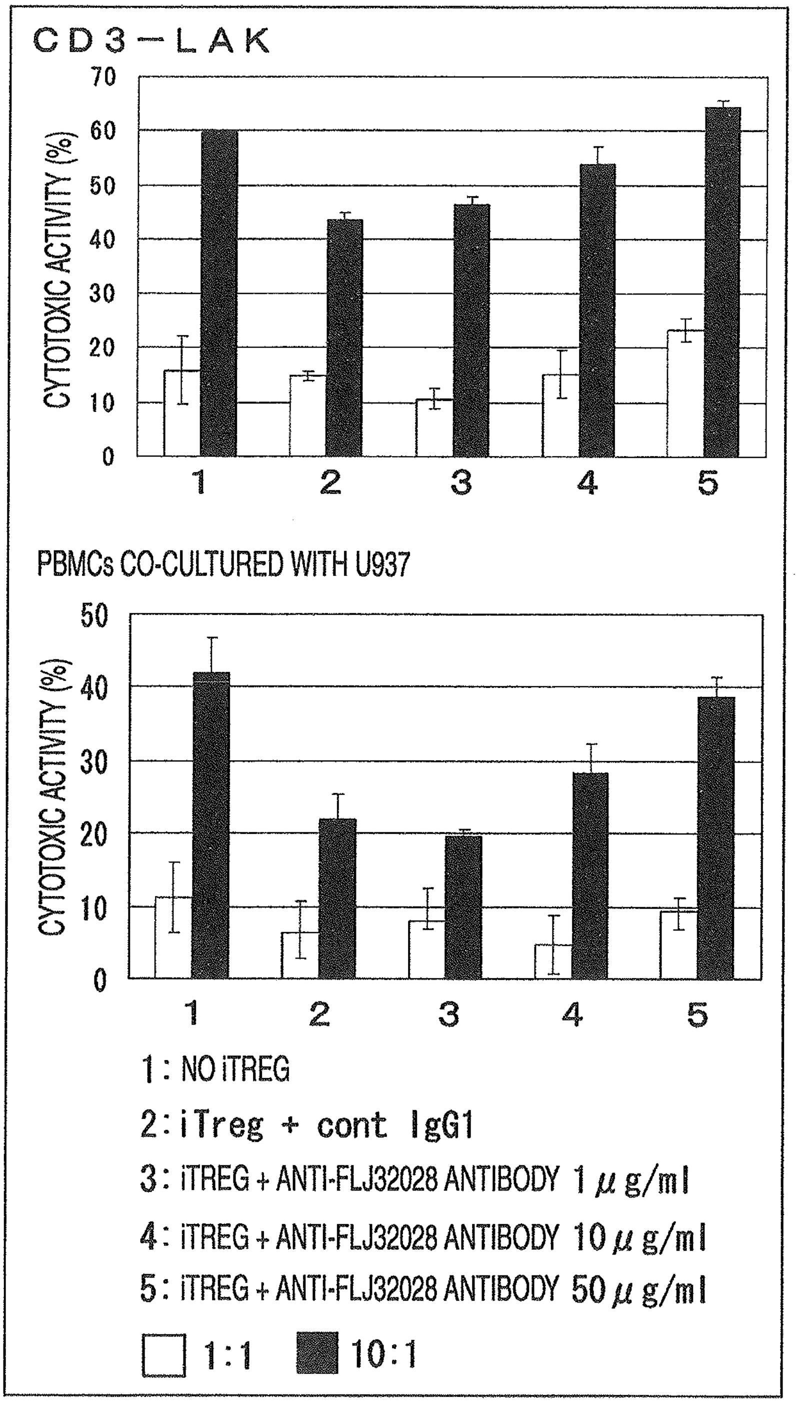
CD3−LAK
CYTOTOXIC ACTIVITY (%)
70
60
50
40
30
20
10
0
1
2
3
4
5
PBMCs CO-CULTURED WITH U937
CYTOTOXIC ACTIVITY (%)
50
40
30
20
10
0
1
2
3
4
5
1: NO iTREG
2: iTreg + cont IgG1
3: iTREG + ANTI-FLJ32028 ANTIBODY 1 μg/ml
4: iTREG + ANTI-FLJ32028 ANTIBODY 10 μg/ml
5: iTREG + ANTI-FLJ32028 ANTIBODY 50 μg/ml
1:1
10:1

FIG.11

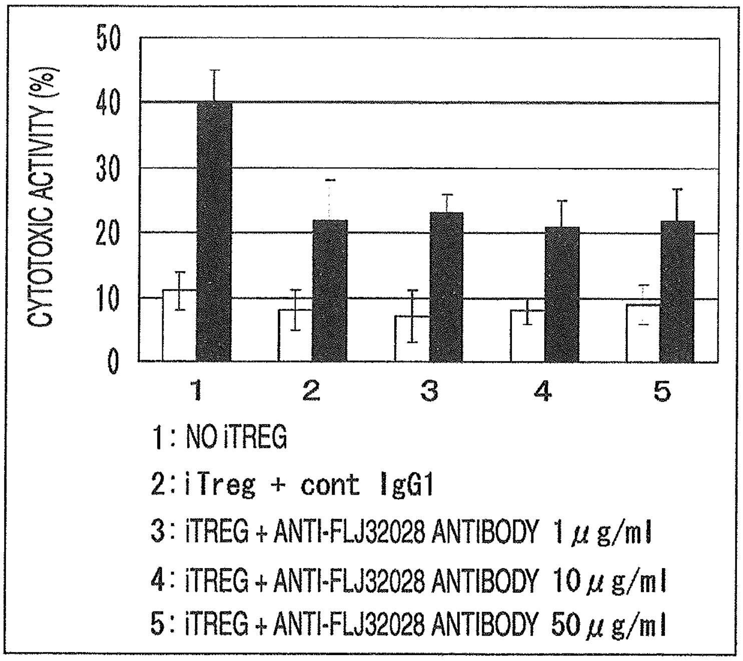
CYTOTOXIC ACTIVITY (%)
0
10
20
30
40
50
1
2
3
4
5
1 : NO iTREG
2 : iTreg + cont IgG1
3 : iTREG + ANTI-FLJ32028 ANTIBODY 1 μg/ml
4 : iTREG + ANTI-FLJ32028 ANTIBODY 10 μg/ml
5 : iTREG + ANTI-FLJ32028 ANTIBODY 50 μg/ml

FIG.12

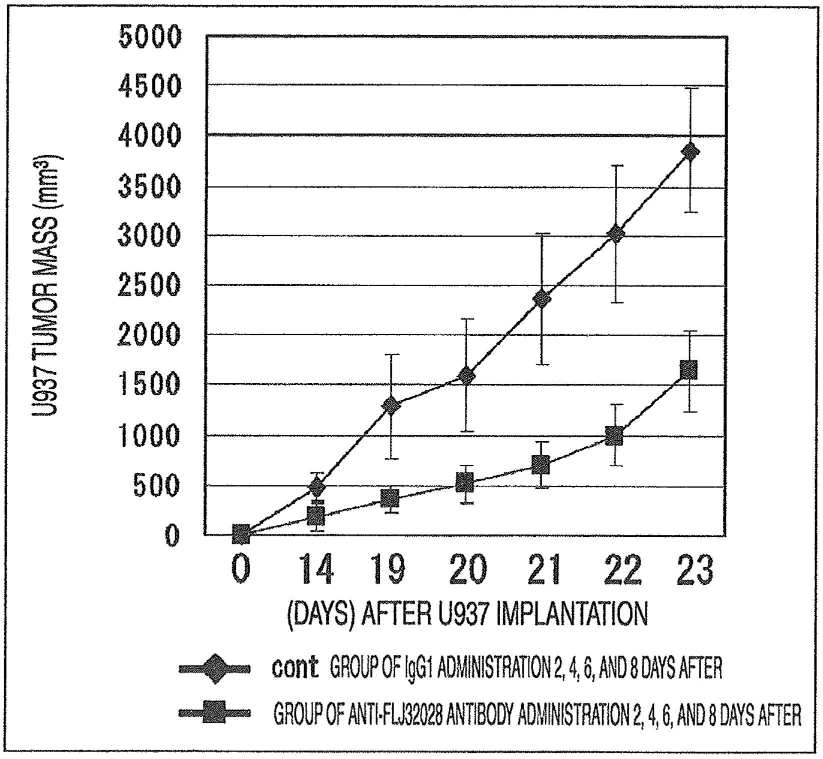
U937 TUMOR MASS (mm³)
0
500
1000
1500
2000
2500
3000
3500
4000
4500
5000
0
14
19
20
21
22
23
(DAYS) AFTER U937 IMPLANTATION
cont GROUP OF IgG1 ADMINISTRATION 2, 4, 6, AND 8 DAYS AFTER
GROUP OF ANTI-FLJ32028 ANTIBODY ADMINISTRATION 2, 4, 6, AND 8 DAYS AFTER

**INTERNATIONAL SEARCH REPORT**

International application No.
PCT/JP2009/054842

A. CLASSIFICATION OF SUBJECT MATTER
*C07K16/28*(2006.01)i, *A61K39/395*(2006.01)i, *C12Q1/04*(2006.01)i, *C12P21/08*(2006.01)n

According to International Patent Classification (IPC) or to both national classification and IPC

B. FIELDS SEARCHED

Minimum documentation searched (classification system followed by classification symbols)
C07K16/28, A61K39/395, C12Q1/04, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAPlus/BIOSIS/MEDLINE/WPIDS(STN), JSTPlus/JMEDPlus/JST7580(JDreamII), GenBank/EMBL/DDBJ/GeneSeq, UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X/A | JP 2007-525195 A (Alexion Pharmaceuticals, Inc.), 06 September, 2007 (06.09.07), Particularly, example 3; Claims 13, 17 to 18; Par. Nos. [0017] to [0021], [0159] to [0167], [0189] & US 2008/0057519 A1 & WO 2004/110369 A2 | 1-6,11,14, 16,17/7-10, 12,13,15,18, 19 |
| A | Toshimasa TADAKI et al., "$CD4^+CD25^+$Treg Saibo Jokyo ni Yuyo na Saibo Hyomen Bunshi Dotei no Tameno cDNA Subtraction-ho to Tokuiteki Kotai no Sakuseiho", Dai 35 Kai Proceedings of the Japanese Society for Immunology, 15 November, 2005 (15.11.05), page 133, 1-I-W17-28-P | 1-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 June, 2009 (02.06.09) | 16 June, 2009 (16.06.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/054842

C (Continuation). DOCUMENTS CONSIDERED TO BE RELEVANT

| Category* | Citation of document, with indication, where appropriate, of the relevant passages | Relevant to claim No. |
|---|---|---|
| A | Hiromichi YAMASHIRO et al., "Ko Shuyo Men'eki Kassei o Zokyo saseru Yokusei Koka Jokyo ni Yuko na Treg Saibo Marker no Tansaku", Dai 35 Kai Proceedings of the Japanese Society for Immunology, 15 November, 2005 (15.11.05), page 133, 1-I-W17-27-O/P | 1-19 |
| A | JP 2006-304740 A (Kyoto University), 09 November, 2006 (09.11.06), Particularly, Par. Nos. [0006] to [0007]; examples 3 to 5 & US 2006/0246063 A1 | 1-19 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004110369 A
- JP 2006304740 A


### Non-patent literature cited in the description


- **Dannull J et al.** *J Clin Invest.,* December 2005, vol. 115 (12), 3623-33
- **Shimizu J et al.** *Nature Immunology,* 01 February 2002, vol. 3, 135-142
- **Phan GQ et al.** *Proc. Natl. Acad. Sci. U.B.A.,* vol. 100, 372
- *Nat. Genet.,* 2004, vol. 36 (1), 40-45
- *J Immunol.,* 05 August 2002, vol. 169 (4), 1893-903
- *Clin Exp Immunol.,* February 1995, vol. 99 (2), 160-7